# EUROPEAN PATENT SPECIFICATION

(11) **EP 2 056 918 B1**
(45) Date of publication and mention of the grant of the patent: **23.08.2017**
(21) Application number: 07837456.8
(22) Date of filing: 29.08.2007
(51) Int. Cl.: A61M 29/00

(54) **CATHETER FOR CELL DELIVERY**
KATHETER FÜR ZELLENFREISETZUNG
CATHÉTER SERVANT À ADMINISTRER DES CELLULES

(30) Priority: 29.08.2006 US 841009 P
(43) Date of publication of application: 13.05.2009
(73) Proprietor: Tissue Genesis, Inc., Honolulu, HI 96813 (US)
(72) Inventor: BOLAND, Eugene D., Honolulu, HI 96825 (US); WILLIAMS, Stuart K., Harrods Creek, KY 40027 (US); KOSNIK, Paul E., Jonestown, TX 78645 (US)
(74) Representative: Roberts, Peter David
(86) International application number: PCT/US2007/018968
(87) International publication number: WO 2008/027416

(56) References cited:
- EP-A2- 0 835 673
- WO-A1-00/41761
- US-A1- 2003 114 793
- US-A1- 2006 079 957
- US-B1- 6 544 223
- US-B2- 6 958 059

## Description

### CROSS-REFERENCE TO RELATED PATENT APPLICATION OR PRIORITY CLAIM

This application claims priority on United States Provisional Patent Application No. 60/841,009, filed August 29, 2006.

### FIELD OF THE INVENTION

The present invention relates generally to balloon catheters and more specifically to double bladder catheters suitable for delivering cells to cylindrical or tubular tissues, body cavities, or joints. More specifically, the present invention relates to localized delivery of cells utilizing a sustained low pressure sodding technique. Such a catheter is disclosed in the EP835673.

### BACKGROUND

Despite the development in recent years of a number of innovative treatments, cardiovascular disease remains a leading cause of debilitation and death worldwide in men and women over the age of sixty-five. In many countries cardiovascular disease is viewed as a "second epidemic," replacing infectious diseases as the leading cause of death.

Endarterectomy, atherectomy, and angioplasty are common surgical procedures used to treat damaged blood vessels and remove plaque (a mixture of fatty substances, including cholesterol and other lipids). Carotid endarterectomy is commonly used to remove plaque buildups in the carotid arteries. During the procedure, a physician makes an incision in the affected artery and removes the plaque contained in the artery's inner lining. Endarterectomy is also used to treat peripheral arterial disease, renal artery disease, aortic arch conditions, aortoiliac occlusive disease, visceral (intestines, spleen, and liver) artery disease.

Atherectomy is a procedure to remove plaque from a blood vessel using a laser catheter, or a rotating shaver ("burr" device on the end of a catheter). The catheter is inserted into the body and advanced through an artery to the area of narrowing. Other devices that can be used are dissectional catheterectomy, catheters that shave off the plaque, or laser catheters that vaporize the plaque. An atherectomy is useful in cases where the plaque is very hard due to calcification, plaque has built up in a coronary artery bypass graft, or to remove of other difficult blockages.

Angioplasty involves the passage of a balloon catheter into the lesion followed by dilatation of the blocked segment. Angioplasty is extensively used to treat carotid lesions, peripheral arterial disease.

Atherectomy and angioplasty may be followed by placement of a stent, which acts as a scaffold to prevent the reclosure of the blood vessel. The stent allows the normal flow of blood and oxygen in the blood vessel. With traditional bare-metal uncoated stents, about 20% of patients who undergo angioplasty experience restenosis (scarring), which can narrow or block the blood vessel again. Use of a drug-coated stent dramatically lowers the patient's risk of needing another procedure due to restenosis. However, a drug-coated stent has a tendency to cause thrombosis (the formation of blood clots inside a stent that can be deadly) because the drug prevents healing around the stent. Anti-thrombotic drugs have been used to counteract this effect. However, anti-thrombotic drugs cause rashes and bleedings, and must be used indefinitely by patients, leading to problems with compliance.

While the short term benefit of these procedures can be dramatic, the procedures disrupt the endothelium, which is the leading cause of restenosis.

### OVERVIEW

A cell delivery system is described comprising a catheter configured to deliver cells in a pressure controlled manner to a tissue or body cavity, as further disclosed in claim 1. In an embodiment, the cell delivery system is used as a primary treatment for stenosis or trauma. In an embodiment, the cell delivery system is used to treat injury caused by prior intervention, including balloon angioplasty, artherectomy, or endarterectomy. In an embodiment, the cell delivery system is used to deliver cells into a body cavity, such as to the heart or a joint.

The catheter may comprise an inner bladder and an outer perforated bladder that permits localized delivery of stem cells. The inner bladder may be expanded through the use of a pressure conduit in order to deploy a stent. Cells, such as endothelial cells derived from adipose tissue, may be introduced between the inner and outer bladder. The inner bladder may be further expanded in order to exert pressure on the outer perforated bladder to advance the cells through the apertures of the outer bladder. The inner bladder may remain pressurized to hold the outer bladder against the vessel wall, thereby directing the cells to specific target sites. The system may be used to deliver cells with or without other therapeutic agents. The cells may comprise stem cells. The apertures may preferably be configured to permit passage of cells and small cell aggregates that are approximately 50 to 100 µm. The catheter may also carry a guide wire in its own added lumen, to facilitate the insertion of the catheter in a manner which is conventional to the clinical catheter art. The stent may be coated to promote cell adhesion. The bladders may be designed to resist abrasion due to stent deployment. The system may further comprise a pressure gauge that permits measurement and regulation of pressure within the bladders.

### BRIEF DESCRIPTION OF THE DRAWINGS

The accompanying drawings, which are incorporated into and constitute a part of this specification, illustrate one or more embodiments and, together with the detailed description, serve to explain the principles and implementations of the invention.
FIG. 1 is a cross-sectional side view of a distal tip of a double bladder catheter implanted in the lumen of a blood vessel.
FIG. 2 is a side view of a cell delivery system implanted into a blood vessel.
FIG. 3a is a side view of a cell delivery system that includes a detachable reservoir with a pressure gauge.
FIG. 3b is a side view of a cell delivery system, wherein a reservoir is detached from the catheter.
FIG. 4 is a side view of a cell delivery system that includes a double lumen catheter.
FIG. 5 is a cross-sectional side view of a dual lumen catheter having tubes that are coaxially mounted.

### DESCRIPTION OF EXAMPLE EMBODIMENTS

Those of ordinary skill in the art will realize that the following detailed description is illustrative only and is not intended to be in any way limiting. Other embodiments will readily suggest themselves to such skilled persons having the benefit of this disclosure.

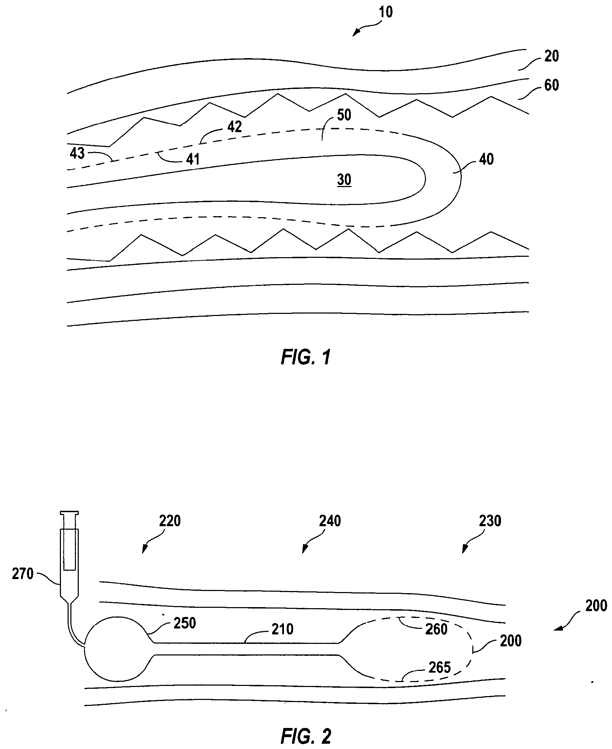

FIG. 1 is a cross-sectional perspective view of a distal tip of a double bladder catheter 10 located in the lumen of a blood vessel 20. The catheter 10 comprises an inner bladder 30 and an outer bladder 40 having a plurality of apertures 50. The inner bladder 30 may be composed of polyurethane, silicone, polyethylene, polycarbonate, or a combination thereof.

The outer bladder 40 may be composed of expanded polytetrafluoroethylene, polyurethane, polypropylene, polyethylene, polyamides, nylon, elastin, polyethylene terephthalate, polycarbonate, silicone, or combinations thereof. The outer bladder 40 may be surface treated to reduce cell attachment. The outer bladder 40 may have a thickness of about 0.051 mm (0.002 inches) to about 2.54mm (0.100 inches), defined by an inner surface 41, outer surface 42, and vertical surface 43. In an embodiment, the inner surface 41, outer surface 42 and vertical surface 43 are surface treated. The outer bladder 40 may comprise hydrophilic material to facilitate cell and fluid transit. The diameter of the apertures 50 of outer bladder 40 may be between about 2 to about 1000 microns to facilitate passage of cells and small cell aggregates through outer bladder 40. The apertures 50 may be sufficiently small so that the outer bladder 40 may be pressure inflated despite the presence of the apertures 50 to permit outer bladder 40 to be held against a stent 60 to deliver cells and other therapeutic agents to the stent 60. The stent 60 may be coated to promote cell adhesion.

The stent 60 may first be deployed in the blood vessel 20. The stent 60 may be deployed by applying pressure to the inner bladder 30. The outer bladder 40 may then be loaded with cells and other therapeutic media. The inner bladder 30 may then be further pressurized to advance the cells and other therapeutic agents through the apertures 50 of the outer bladder 40 in order to introduce cells to the stent 60 or other device.

FIG. 2 is a side view of a cell delivery device 200 located into a blood vessel 205. The cell delivery device 200 may comprise a catheter 210 comprising a proximal end 220 and a distal end 230, and defining a lumen 240 therebetween. The proximal end 220 may comprise a fluid reservoir 250, which may be filled with a fluid carrier, cells, and other therapeutic agents. The distal end 230 may comprise a bladder 260 having a plurality of apertures 265.

A pressure conduit 270 may engage the liquid reservoir 250 to increase the pressure within liquid reservoir 250. The increased pressure may advance the contents of the liquid reservoir 250 into the lumen 240 of the catheter 210 and into the bladder 260. Application of further pressure may inflate bladder 260 so that it contacts the lumen surface of the blood vessel 205 and advances the cells, fluid, and other therapeutic agents through the apertures of the bladder 260 to targeted sites. The pressure conduit 270 may maintain pressure on the bladder 260, maintaining a pressure gradient against the lumenal surface of the blood vessel and permitting cells and other therapeutic agents to transmit the lumen 240 of the catheter 210 to the lumen surface of the blood vessel 205. Removal of pressure from the lumen 240 may result in deflation of the bladder 260.

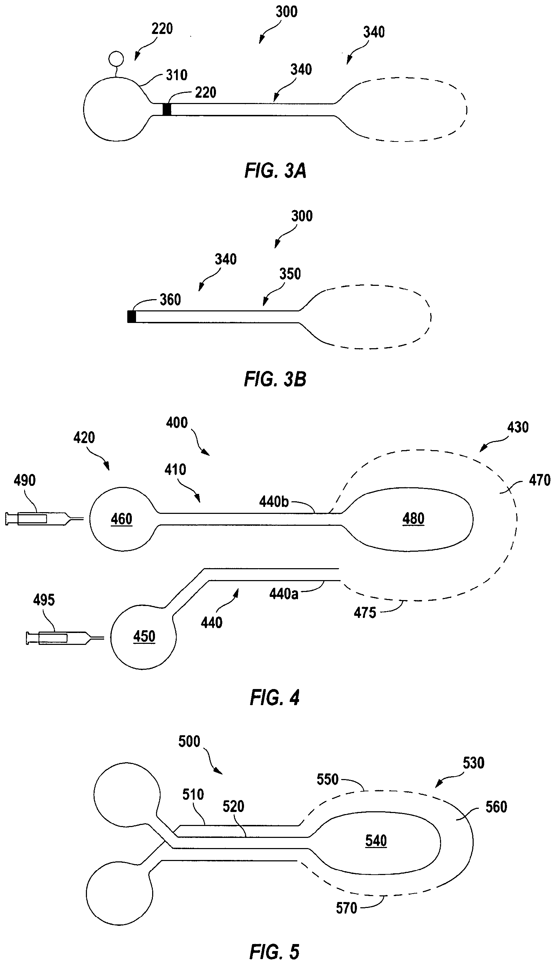

FIG. 3a is a side view of a cell delivery device 300 that includes a reservoir 310 that includes a pressure gauge 320. The reservoir 310 may be removably attached to a lumen 340 of a catheter 350. The lumen 350 may further comprise a valve 360. The pressure gauge 320 may be used to measure the pressure of the reservoir 310. The pressure gauge 320 may communicate, either automatically or with human intervention, with a pressure conduit 370 to maintain the pressure of the reservoir within specified parameters. Pressure may be maintained between 7N/m² and 170,000N/m² (0.001 PSI and 25 PSI) depending upon the application.

FIG. 3b is a side view of a cell delivery system 300, wherein the reservoir 310 (not shown) is detached from the lumen 340 of a catheter 350. In an embodiment, valve 360 is used to close the posterior end of lumen 340 prior to the detachment of reservoir 310 so that the pressure may be maintained within the lumen 340 of the catheter 350.

FIG. 4 is a side view of a cell delivery system 400. The cell delivery system 400 may comprise a catheter 410 comprising a proximal end 420 and a distal end 430 defining a lumen 440 therebetween. The proximal end 420 may comprise a fluid reservoir 450 and a pressure reservoir 460. The distal end 430 may comprise an outer porous bladder or sheath 470, having a plurality of apertures 475, and a non-porous inner bladder 480.

The lumen 440 may be a dual lumen, comprising a first tube 440a between the liquid reservoir 450 and the outer bladder 430 and a second tube 440b between the pressure reservoir 460 and the inner bladder 480.

A first pressure conduit 490 may engage the pressure reservoir 460 to increase the pressure within pressure reservoir 460. The increased pressure may advance the contents of the pressure reservoir 460 into the second tube 440b of the lumen 440 of the catheter 410 and into the inner bladder 480. Cells and other therapeutic agents may then be loaded into the liquid reservoir 450. Alternatively, the cells and other therapeutic agents may be preloaded into the liquid reservoir 450.

A second pressure conduit 495 may be used to apply a pressure to the liquid reservoir 450, advancing the liquid carrier, cells, and other therapeutic agents into the first tube 440a of the lumen 440 of the catheter 410 and then into the outer bladder 470. In an embodiment, the first pressure conduit 490 is the same as the second pressure conduit 495. In this embodiment, the contents of the pressure conduit when used to increase the pressure of the liquid reservoir may be the same or be different than the contents of the pressure conduit when used to apply pressure to the pressure reservoir.

The first pressure conduit 490 may then further pressurize the inner bladder 480, which exerts pressure on the outer bladder and advances the cells and other therapeutic agents out of the outer bladder 470 through the apertures 475.

FIG. 5 is a cross-sectional side view of a catheter 500 configured to deliver cells to the lumenal surface of a tubular tissue comprising coaxially mounted dual lumen tubes 510 and 520 that are attached to a double layered balloon 530. The double layered balloon 530 has an inner chamber 540 concentrically positioned within an outer chamber 550 in a spaced apart relationship defining an annular lumen 560 therebetween. The outer chamber 550 comprises a plurality of apertures 570. Cells may be disposed within the annular lumen 560 and delivered to the lumenal surface of a tubular tissue through the apertures 570 of the outer chamber 550.

Specific examples of cells that may be used include cells that are derived from adipose tissue, such as endothelial cells and growth factor producing cells; cells that are derived from bone marrow, such as mesenchymal cells; cells that are derived from blood, such as endothelial progenitor cells; cells derived from fetal tissue; cells that are derived from skeletal muscle; cells derived from an umbilical cord; cells that are genetically modified to produce a protein product, such as factor VIII, a protein involved in the blood-clotting process lacked by some hemophiliacs, and insulin, a protein hormone that regulates blood glucose levels. Adipose derived endothelial cells are pluripotent stem cells, having the ability to differentiate into smooth muscle or other types of cells, as described in Oliver Kocher and Joseph A. Madri, Modulation of Actin mRNAs in Cultured Vascular Cells By Matrix Components and TGF-β, In Vitro Cellular & Developmental Biology, Vol. 25, No. 5. May 1989.

Cells that are encapsulated to allow cells to secrete hormones or provide a specific metabolic function without being recognized by the immune system may be used. As such, they can be implanted without rejection. Cells that are genetically engineered to express a naturally occurring protein that disables immune system cells that bind to it may also be used.

Therapeutic agents may include Transforming Growth Factor beta (TGF*β*) and TGF-*β*-related proteins for regulating stem cell renewal and differentiation.

Therapeutic agents that may be used include angiogenesis-related cytokines, such as vascular endothelial growth factor (VEGF) and hepatocyte growth factor (HGF), anti-thrombogenic agents or other agents for suppressing stenosis or late restenosis such as heparin, streptokinase, urokinase, tissue plasminogen activator, anti-thromboxane B² agents, anti-B-thromboglobulin, prostaglandin E, aspirin, dipyridimol, anti-thromboxane A₂ agents, murine monoclonal antibody 7E3, triazolopyrimidine, ciprostene, hirudin, ticlopidine, nicorandil, and the like. Anti-platelet derived growth factor may be used as a therapeutic agent to suppress subintimal fibromuscular hyperplasia at an arterial stenosis site, or any other inhibitor of cell growth at the stenosis site may be used.

Other therapeutic agents that may be used in conjunction with stem cells may comprise a vasodilator to counteract vasospasm, for example an antispasmodic agent such as papaverine. The therapeutic agents may be vasoactive agents generally such as calcium antagonists, or alpha and beta adrenergic agonists or antagonists. Additionally, the therapeutic agent may include a biological adhesive such as medical grade cyanoacrylate adhesive or fibrin glue, for example to adhere an occluding flap of tissue in a coronary artery to the wall, or for a similar purpose. Additionally, the therapeutic agent may be an anti-neoplastic agent such as 5-fluorouracil or any known anti-neoplastic agent, preferably mixed with a controlled release carrier for the agent, for the application of a persistent, controlled release anti-neoplastic agent to a tumor site.

The therapeutic agent may be an antibiotic, which may be applied to an infected stent or any other source of localized infection within the body. Similarly, the therapeutic agent may comprise steroids for the purpose of suppressing inflammation or for other reasons in a localized tissue site.

Additionally, glucocorticosteroids or omega-3 fatty acids may be applied, particularly to stenosis sites. Any of the therapeutic agents may include controlled release agents to prolong the persistence.

The therapeutic agent may constitute any desired mixture of individual pharmaceuticals or the like, for the application of combinations of active agents. The pharmaceutical agent may support the survival of the cell (e.g., a carbohydrate, a cytokine, a vitamin, etc.).

Cells can be delivered with a pharmaceutically acceptable carrier. Examples of pharmaceutically acceptable carriers include excipients, lubricants, binders, disintegrants, disintegration inhibitors, absorption promoters, adsorbers, moisturizing agents, solvents, solubilizing agents, suspending agents, isotonic agents, buffers, soothing agents and the like. Additives for formulations, such as antiseptics, antioxidants, colorants, and the like can be optionally used.

Combinations may be administered either concomitantly (e.g., as an admixture), separately but simultaneously or concurrently; or sequentially. This includes presentations in which the combined agents are administered together as a therapeutic mixture, and also procedures in which the combined agents are administered separately but simultaneously. "Combination" administration further includes the separate administration of one of the compounds or agents given first, followed by the second.

Formulation materials or pharmaceutically acceptable agents that may be used include, but are not limited to, antioxidants, preservatives, coloring, and diluting agents, emulsifying agents, suspending agents, solvents, fillers, bulking agents, buffers, delivery vehicles, diluents, excipients and/or pharmaceutical adjuvants. Representatively, a medicament may be administered in the form of a composition additionally comprising an active ingredient (e.g., a cell), at least one physiologically acceptable carrier, an excipient, or a diluent. For example, a suitable vehicle may be water for injection, physiological saline solution, or artificial cerebrospinal fluid.

Acceptable carriers, excipients or stabilizers used herein may be nontoxic to recipients and inert at the dosages and concentrations employed, and may include buffers such as phosphate, citrate, or other organic acids; ascorbic acid, *α*-tocophenol; low molecular weight polypeptides; proteins (e.g., serum albumin, gelatin, or immunoglobulins); hydrophilic polymers (e.g., polyvinylpyrrolidone); amino acids (e.g., glycine, glutamine, asparagine, arginine or lysine); monosaccharides, disaccharides, and other carbohydrates (including glucose, mannose, or dextrins); chelating agents (e.g., EDTA); sugar alcohols (e.g., mannitol or sorbitol); salt-forming counterions (e.g., sodium); and/or nonionic surfactants (e.g., Tween, pluronics or polyethylene glycol (PEG)).

Neutral buffered saline or saline mixed with serum albumin are exemplary appropriate carriers. The product may be formulated as a lyophilizate using appropriate excipients (e.g., sucrose). Other standard pharmaceutically acceptable carriers, diluents, and excipients may be included as desired. Other exemplary compositions comprise Tris buffer of about pH 7.0-8.5, or acetate buffer of about pH 4.0-5.5, which may further include sorbitol or a suitable substitute therefor.

Examples of excipients include glucose, lactose, sucrose, D-mannitol, crystallized cellulose, starch, calcium carbonate, light silicic acid anhydride, sodium chloride, kaolin, urea, and the like.

Examples of absorption promoters include, but are not limited to, quaternary ammonium salts, sodium lauryl sulfate, and the like.

Examples of stabilizers include, but are not limited to, human serum albumin, lactose, and the like.

Examples of suspending agents in liquid formulations include surfactants (e.g., stearyltriethanolamine, sodium lauryl sulfate, lauryl amino propionic acid, lecithin, benzalkonium chloride, benzethonium chloride, glycerin monostearate, etc.), hydrophilic macromolecule (e.g., polyvinyl alcohol, polyvinylpyrrolidone, carboxymethylcellulose sodium, methylcellulose, hydroxymethylcellulose, hydroxyethylcellulose, hydroxyethylcellulose, hydroxypropylcellulose, etc.), and the like.

Examples of solvents in liquid formulations include injection solutions, alcohols, propyleneglycol, macrogol, sesame oil, corn oil, and the like.

Examples of solubilizing agents in liquid formulations include, but are not limited to, polyethyleneglycol, propyleneglycol, D-mannitol, benzyl benzoate, ethanol, trisaminomethane, cholesterol, triethanolamine, sodium carbonate, sodium citrate, and the like.

Examples of isotonic agents in liquid formulations include, but are not limited to, sodium chloride, glycerin, D-mannitol, and the like.

Examples of buffers in liquid formulations include, but are not limited to, phosphate, acetate, carbonate, citrate, and the like.

Examples of soothing agents in liquid formulations include, but are not limited to, benzyl alcohol, benzalkonium chloride, procaine hydrochloride, and the like.

Examples of antiseptics in liquid formulations include, but are not limited to, parahydroxybenzoate esters, chlorobutanol, benzyl alcohol, 2-phenylethylalcohol, dehydroacetic acid, sorbic acid, and the like.

Examples of antioxidants in liquid formulations include, but are not limited to, sulfite, ascorbic acid, *α*-tocopherol, cysteine, and the like.

Liquid agents may be sterilized and may be isotonic with the blood or a medium at a target site. Typically, these agents are made aseptic by filtration using a bacteria-retaining filter or the like, mixing with a bactericide or, irradiation, or the like. Following this treatment, these agents may be made solid by lyophilization or the like. Immediately before use, sterile water or sterile injection diluent (lidocaine hydrochloride aqueous solution, physiological saline, glucose aqueous solution, ethanol or a mixture solution thereof, etc.) may be added.

The liquid carrier used may be in the form of a pyrogen-free, pharmaceutically acceptable aqueous solution. The preparation of such pharmaceutically acceptable compositions, with due regard to pH, isotonicity, stability and the like, is within the skill of the art.

As used herein, the term "pressure conduit" refers to a means which may be in communication with a reservoir and is used for adjusting the pressure applied to the cell delivery system. The pressure conduit may be a syringe. The cell delivery system may be constructed so that a liquid carrier containing cells may be pressurized within a predetermined pressure range, which may be between 7N/m² to 170,000 N/m² (.001 PSI and 25 PSI).

The pressure can be adjusted manually or automatically. With automatic control, it is possible to suppress a sudden change in pressure which may occur in manual control.

The medical device may be particularly useful for treatment of diseased tissues after rotablation, angioplasty, stent placement, bypass graft implantation-both natural and synthetic.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described
herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

## Claims

1. A cell delivery system (200) for localized delivery of cells, the cell delivery system comprising a tube (210) with a proximal end (220) and a distal end (230) defining a lumen (240) therebetween containing cells and fluid, the distal end (230) configured to deliver cells to a tubular tissue in a pressure controlled manner,
wherein the distal end (230) comprises a sheath comprising a plurality of apertures (265) configured to permit the passage of cells and small cell aggregates that are approximately 50 to 100 µm,
the system further comprises a pressure conduit (270) to apply and sustain a pressure in the interior of the sheath (260) such that a pressure gradient is maintained between the lumen (240) and a luminal surface of the tubular tissue, whereby the cells are delivered to the luminal surface through the apertures (265) of the sheath (260), said sustained pressure being between about 7 N/m² (0.001 PSI) to about 170,000 N/m² (25 PSI).

2. The system of claim 1, wherein the sheath (260) comprises a material selected from the group consisting of polytetrafluoroethylene, expanded polytetrafluoroethylene, polyurethane, polypropylene, polyethylene, polyamides, nylon, elastin, polyethyleneterephthalate, polycarbonate, silicone, and combinations thereof.

3. The system of claim 1, wherein the sheath (260) comprises an outer surface, inner surface, and a vertical surface that are surface treated to reduce cell adhesion.

4. The system of claim 1, wherein the sheath is hydrophilic.

5. The system of claim 1, wherein the cells are chosen into the group consisting in cells derived from adipose tissue, mesenchymal stem cells, cells derived from bone marrow, cells derived from blood, endothelial cells derived from adipose tissue and stem cells.

6. The system of claim 1, wherein the sheath (260) is configured to expand upon application of pressure from the pressure conduit (270).

7. The system of claim 1, wherein the sheath (260) is configured to deflate upon removal of pressure or upon removal of pressure from the pressure conduit (270).

8. The system of claim 1, wherein the pressure conduit (270) is a syringe.

9. The system of claim 1, wherein the proximal end further comprises a pressure reservoir (460) and the distal end (430) of the catheter (410) further comprises an inner bladder (480), and wherein the pressure conduit (460) is a tube that passes through the lumen.

10. The system of any one of claim 1 or 9, wherein the fluid reservoir (450) or the pressure reservoir (460) comprises a pressure gauge (320).

11. The system of claim 1, further comprising a fluid reservoir (450) connected to the sheath through a passage within the lumen (440) outside of the pressure conduit (490).

12. The system of any of claim 9 or 11, wherein the fluid reservoir (450) or the pressure reservoir (490) is removably attached to the catheter (410).

13. The system of claim 12, wherein the system is configured to maintain pressure on the sheath upon removal of the fluid reservoir (450).

14. The system of claim 12, wherein the system is configured to maintain pressure on the inner bladder (480) upon removal of the pressure reservoir (490).

15. The system of claim 9, wherein the pressure conduit tube (490) is contained concentrically within the space defined by the lumen (440) and is contiguous with the inner bladder (480).

## Patentansprüche

1. System (200) zur lokalisierten Zellenfreisetzung, wobei das Zellenfreisetzungssystem ein Rohr (210) mit einem proximalen Ende (220) und einem distalen Ende (230) umfasst, welche ein Lumen (240) dazwischen definieren, welches Zellen und Fluid umfasst, wobei das distale Ende (230) konfiguriert ist, um Zellen in druckgelenkter Weise an ein rohrförmiges Gewebe abzugeben,
wobei das distale Ende (230) eine Hülse umfasst, welche eine Vielzahl von Öffnungen (265) umfasst, die konfiguriert sind, um das Durchleiten von Zellen und kleinen Zellaggregaten einer Größe von ungefähr 50 bis 100 µm zu ermöglichen,
wobei das System zudem eine Druckleitung (270) umfasst, um einen Druck auf das Innere der Hülse (260) auszuüben und zu halten, sodass ein Druckgradient zwischen dem Lumen (240) und einer luminalen Fläche des rohrförmigen Gewebes gehalten wird wobei die Zellen durch die Öffnungen (265) der Hülse (260) freigesetzt werden, wobei der gehaltene Druck zwischen ungefähr 7 N/m² (0,001 PSI) und ungefähr 170.000 N/m² (25 PSI) liegt.

2. System nach Anspruch 1, bei welchem die Hülse (260) ein Material umfasst, gewählt aus der Gruppe, bestehend aus Polytetrafluorethylen, expandiertem Polytetrafluorethylen, Polyurethan, Polypropylen, Polyethylen, Polyamiden, Nylon, Elastin, Polyethylen-Terephthalat, Polycarbonat, Silikon, und Kombinationen davon.

3. System nach Anspruch 1, bei welchem die Hülse (260) eine äußere Fläche, eine innere Fläche und eine vertikale Fläche umfasst, welche oberflächenbehandelt sind, um die Zellanhaftung zu reduzieren.

4. System nach Anspruch 1, bei welchem die Hülse hydrophil ist.

5. System nach Anspruch 1, bei welchem die Zellen in der Gruppe, bestehend aus Zellen aus Fettgewebe, mesenchymalen Stammzellen, Zellen aus Knochenmark, Zellen aus Blut, Endothelzellen aus Fettgewebe und Stammzellen gewählt werden.

6. System nach Anspruch 1, bei welchem die Hülse (260) konfiguriert ist, um bei Ausüben von einem Druck aus der Druckleitung (270) zu expandieren.

7. System nach Anspruch 1, bei welchem die Hülse (260) konfiguriert ist, um sich bei Entfernen von einem Druck aus der Druckleitung (270) zu entleeren.

8. System nach Anspruch 1, bei welchem die Druckleitung (270) eine Spritze ist.

9. System nach Anspruch 1, bei welchem das proximale Ende zudem einen Drucktank (460) umfasst und das distale Ende (430) des Katheters (410) zudem eine innere Blase (480) umfasst, und wobei die Druckleitung (460) ein Rohr ist, das durch das Lumen hindurchführt.

10. System nach einem der Ansprüche 1 oder 9, bei welchem der Fluidtank (450) oder der Drucktank (460) einen Druckmesser (320) umfasst.

11. System nach Anspruch 1, zudem umfassend einen Fluidtank (450), welcher mit der Hülse durch eine Durchführung innerhalb des Lumens (440) außerhalb der Druckleitung (490) verbunden ist.

12. System nach einem der Ansprüche 9 oder 11, bei welchem der Fluidtank (450) oder der Drucktank (490) abnehmbar am Katheter (410) befestigt ist.

13. System nach Anspruch 12, bei welchem das System konfiguriert ist, um einen Druck auf die Hülse nach Entfernen des Fluidtanks (450) zu halten.

14. System nach Anspruch 12, bei welchem das System konfiguriert ist, um einen Druck auf die innere Blase (480) nach Entfernen des Drucktanks (490) zu halten.

15. System nach Anspruch 9, bei welchem das Druckleitungsrohr (490) konzentrisch innerhalb des vom Lumen (440) definierten Raumes enthalten ist und an der inneren Blase (480) benachbart ist.

## Revendications

1. Système de délivrance de cellules (200) pour la délivrance localisée de cellules, le système de délivrance de cellules comprenant un tube (210) muni d'une extrémité proximale (220) et d'une extrémité distale (230) qui définissent une lumière (240) entre elles qui contient des cellules et un fluide, l'extrémités distale (230) étant configurée de manière à délivrer des cellules à un tissu tubulaire d'une manière contrôlée par pression, dans lequel :
l'extrémité distale (230) comprend une gaine qui comprend une pluralité d'ouvertures (265) qui sont configurées de manière à permettre le passage de cellules et de petits agrégats de cellules d'approximativement 50 à 100 µm,
le système comprend en outre un conduit de pression (270) pour appliquer et soutenir une pression dans l'intérieur de la gaine (260) de telle sorte qu'un gradient de pression soit maintenu entre la lumière (240) et une surface luminale du tissu tubulaire, les cellules étant délivrées sur la surface luminale au travers des ouvertures (265) de la gaine (260), ladite pression soutenue étant entre environ 7 N/m² (0,001 PSI) et environ 170 000 N/m² (25 PSI).

2. Système selon la revendication 1, dans lequel la gaine (260) comprend un matériau qui est sélectionné parmi le groupe qui est constitué par le polytétrafluoroéthylène, le polytétrafluoroéthylène expansé, le polyuréthane, le polypropylène, le polyéthylène, les polyamides, le nylon, l'élastine, le téréphtalate de polyéthylène, le polycarbonate, le silicone et des combinaisons de ceux-ci.

3. Système selon la revendication 1, dans lequel la gaine (260) comprend une surface externe, une surface interne et une surface verticale qui sont traitées en surface de manière à réduire l'adhérence des cellules.

4. Système selon la revendication 1, dans lequel la gaine est hydrophile.

5. Système selon la revendication 1, dans lequel les cellules sont choisies à l'intérieur du groupe qui est constitué par des cellules dérivées à partir d'un tissu adipeux, des cellules souches mésenchymateuses, des cellules dérivées à partir de la moelle épinière, des cellules dérivées à partir du sang, des cellules endothéliales dérivées à partir d'un tissu adipeux et des cellules souches.

6. Système selon la revendication 1, dans lequel la gaine (260) est configurée de manière à se dilater suite à l'application d'une pression qui provient du conduit de pression (270).

7. Système selon la revendication 1, dans lequel la gaine (260) est configurée de manière à se dégonfler suite à l'enlèvement d'une pression ou suite à l'enlèvement d'une pression qui provient du conduit de pression (270).

8. Système selon la revendication 1, dans lequel le conduit de pression (270) est une seringue.

9. Système selon la revendication 1, dans lequel l'extrémité proximale comprend en outre un réservoir de pression (460) et l'extrémité distale (430) du cathéter (410) comprend en outre une poche interne (480), et dans lequel le conduit de pression (460) est un tube qui passe au travers de la lumière.

10. Système selon soit la revendication 1, soit la revendication 9, dans lequel le réservoir de fluide (450) ou le réservoir de pression (460) comprend un manomètre (320).

11. Système selon la revendication 1, comprenant en outre un réservoir de fluide (450) qui est connecté à la gaine par l'intermédiaire d'un passage à l'intérieur de la lumière (440) à l'extérieur du conduit de pression (490).

12. Système selon soit la revendication 9, soit la revendication 11, dans lequel le réservoir de fluide (450) ou le réservoir de pression (490) est fixé de manière amovible au cathéter (410).

13. Système selon la revendication 12, dans lequel le système est configuré de manière à maintenir une pression sur la gaine suite à l'enlèvement du réservoir de fluide (450).

14. Système selon la revendication 12, dans lequel le système est configuré de manière à maintenir une pression sur la poche interne (480) suite à l'enlèvement du réservoir de pression (490).

15. Système selon la revendication 9, dans lequel le tube de conduit de pression (490) est contenu de manière concentrique à l'intérieur de l'espace qui est défini par la lumière (440) et est contigu à la poche interne (480).
